(19) Europäisches Patentamt
European Patent Office
Office européen des brevets

(11) Veröffentlichungsnummer: **0 027 977**
**A1**

(12) **EUROPÄISCHE PATENTANMELDUNG**

(21) Anmeldenummer: **80106356.1**

(22) Anmeldetag: **18.10.80**

(51) Int. Cl.³: **C 07 D 285/12**, A 61 K 31/41

(30) Priorität: **26.10.79 DE 2943406**

(43) Veröffentlichungstag der Anmeldung: **06.05.81 Patentblatt 81/18**

(84) Benannte Vertragsstaaten: **AT BE CH DE FR GB IT LI LU NL SE**

(71) Anmelder: **BASF Aktiengesellschaft, Carl-Bosch-Strasse 38, D-6700 Ludwigshafen (DE)**

(72) Erfinder: **Frickel, Fritz-Frieder, Dr., Silvanerweg 7, D-6705 Deidesheim (DE)**
Erfinder: **Thieme, Peter C., Dr., Dr.-Hans-Hoffmann-Strasse 5, D-6706 Wachenheim (DE)**
Erfinder: **Franke, Albrecht, Dr., Mandelring 11, D-6706 Wachenheim (DE)**
Erfinder: **Hagen, Helmut, Dr., Max-Slevogt-Strasse 17E, D-6710 Frankenthal (DE)**
Erfinder: **Lenke, Dieter, Dr., Kekuleplatz 1, D-6700 Ludwigshafen (DE)**
Erfinder: **Gries, Josef, Dr., Roemerweg 43, D-6706 Wachenheim (DE)**

(54) **Aminoderivate des 2-Methyl-5-(2-hydroxystyryl)-1,3,4-thiadiazols, Verfahren zu ihrer Herstellung und diese enthaltende Mittel.**

(57) Die vorliegende Erfindung betrifft neue Aminopropanolderivate des 2-Methyl-5-(2-hydroxystyryl)-1,3,4-thiadiazols und ihre Säureadditionssalze, ihre Herstellung und diese Verbindungen enthaltende pharmazeutische Zubereitungen und ihre Verwendung als Arzneimittel bei der Behandlung der coronaren Herzkrankheit, der Hypertonie und von Herzrhythmusstörungen.

EP 0 027 977 A1

ACTORUM AG

0027977

BASF Aktiengesellschaft O.Z. 0050/034119

Aminoderivate des 2-Methyl-5-(2-hydroxystyryl)-1,3,4-thiadiazols, Verfahren zu ihrer Herstellung und diese enthaltende Mittel

Die vorliegende Erfindung betrifft neue Aminopropanolderivate des 2-Methyl-5-(2-hydroxystyryl)-1,3,4-thiadiazols und ihre Säureadditionssalze, ihre Herstellung und diese Verbindungen enthaltende pharmazeutische Zubereitungen und ihre Verwendung als Arzneimittel bei der Behandlung der coronaren Herzkrankheit, der Hypertonie und von Herzrhythmusstörungen.

Es wurde gefunden, daß Verbindungen der allgemeinen Formel (I)

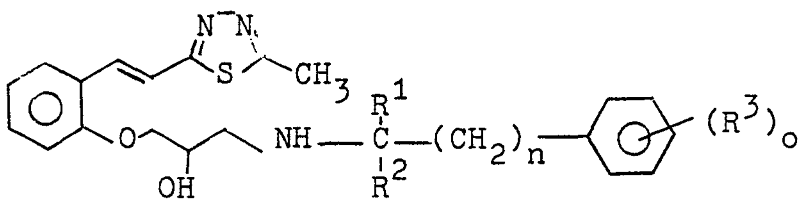 (I),

in der n die Zahlen 2 oder 3, o die Zahlen 1 bis 3, $R^1$ und $R^2$ ein Wasserstoffatom oder eine geradkettige oder verzweigte Alkylgruppe mit 1 bis 5 C-Atomen, $R^3$ ein Wasserstoffatom, ein Halogenatom, eine Hydroxygruppe, eine Alkyl-, Alkoxy- oder Alkylthiogruppe mit 1 bis 5 C-Atomen, gegebenenfalls jeweils in der Alkylgruppe 1- bis 3-fach durch Halogenatome oder einfach durch eine Hydroxy- oder Alkoxygruppe mit 1 bis 3 C-Atomen substituiert, eine Alkenyl-, Alkinyl-, Alkinyloxy- oder Cycloalkoxygruppe mit jeweils 2 bis 6 Kohlenstoffatomen und 3 bis 8 C-Atomen im Ring, eine Aralkoxygruppe mit 7 bis 9 C-Atomen oder eine Aminogruppe, gegebenenfalls mit ein- oder zweifach mit einer Alkylgruppe mit 1 bis 5 Kohlenstoffatomen substituiert, bedeuten, wobei entsprechend den Bedeutungen von o mehrere Reste $R^3$ gleich oder verschieden sein können, oder $R^3$ bedeutet eine Methylendioxygruppe oder eine Alkylengruppe

D/BL

BAD ORIGINAL

mit 3 oder 4 Kohlenstoffatomen, und ihre Säureadditionssalze wertvolle pharmakologische Eigenschaften aufweisen.

Für $R^3$ sind beispielsweise Halogenatome, Fluor und Chlor, gegebenenfalls substituierte Alkyl-, Alkoxy- oder Alkylthiogruppen, Methyl, Äthyl, Propyl, n-Butyl, tert.-Butyl, Methoxy, Äthoxy, Propoxy, Trifluormethyl, Hydroxymethyl, 1-Hydroxyäthyl, Methoxymethyl, Äthoxymethyl, 1-Methoxyäthyl oder n-Propoxymethyl, ungesättigte Reste, beispielsweise Vinyl, Allyl, Propargyloxy, Cycloalkoxyreste, Cyclopentyloxy oder Cyclohexyloxy und Aralkoxyreste Benzyloxy.

Wenn $R^3$ eine Trimethylen- oder Tetramethylengruppe bedeutet, handelt es sich um ein Indanyl- oder Tetrahydronaphtylderivat.

Von den genannten Bedeutungen sind für $R^1$ und $R^2$ Wasserstoff und Methyl und $R^3$ Wasserstoff, Chlor, Trifluormethyl, Alkyl mit 1 bis 4 C-Atomen, Methoxy, Hydroxy, Benzyloxy, wobei mehrere Reste $R^3$ gleich oder verschieden sein können, hervorzuheben.

Demgemäß sind als erfindungsgemäße Verbindungen beispielsweise zu nennen:

2-Methyl-5-[2-[2-hydroxy-3-(2-(3,4-dimethoxyphenyl)-1-methyläthylamino)-propoxy]-styryl]-1,3,4-thiadiazol
2-Methyl-5-[2-[2-hydroxy-3-(3-phenyl-1-methylpropylamino)-propoxy]-styryl]-1,3,4-thiadiazol
2-Methyl-5-[2-[2-hydroxy-3-(3-(4-hydroxyphenyl)-1-methyläthylamino)-propoxy]-styryl]-1,3,4-thiadiazol
2-Methyl-5-[2-[2-hydroxy-3-(3-(4-benzyloxyphenyl)-1-methyläthylamino)-propoxy]-styryl]-1,3,4-thiadiazol
2-Methyl-5-[2-[2-hydroxy-3-(3-(4-hydroxyphenyl)-1,1-dimethylpropylamino)-propoxy]-styryl]-1,3,4-thiadiazol

2-Methyl-5-[2-[2-hydroxy-3-(3-(4-methoxyphenyl)-1,1-dimethylpropylamino)-propoxy]-styryl]-1,3,4-thiadiazol

2-Methyl-5-[2-[2-hydroxy-3-(2-(4-chlorphenyl)-1,1-dimethyläthylamino)-propoxy]-styryl]-1,3,4-thiadiazol

2-Methyl-5-[2-[2-hydroxy-3-(2-(3-trifluormethylphenyl)-1,3-dimethyläthylamino)-propoxy]-styryl]-1,3,4-thiadiazol

2-Methyl-5-[2-[2-hydroxy-3-(2-(4-hydroxyphenyl)-äthylamino)-propoxy]-styryl]-1,3,4-thiadiazol

2-Methyl-5-[2-[2-hydroxy-3-(2-(4-methoxyphenyl)-1,1-dimethyläthylamino)-propoxy]-styryl]-1,3,4-thiadiazol

2-Methyl-5-[2-[2-hydroxy-3-(2-(2-methoxyphenyl)-1,1-dimethyläthylamino)-propoxy]-styryl]-1,3,4-thiadiazol

2-Methyl-5-[2-[2-hydroxy-3-(2-(4-hydroxyphenyl)-1,1-dimethyläthylamino)-propoxy]-styryl]-1,3,4-thiadiazol

2-methyl-5-[2-[2-hydroxy-3-(3-(4-hydroxy-3-methoxyphenyl)-1-methylpropylamino)-propxy]-styryl]-1,3,4-thiadiazol

2-Methyl-5-[2-[2-hydroxy-3-(3-(4-hydroxy-2-methyl-5-tert.butylphenyl)-1-methylpropylamino)-propoxy]-styryl]-1,3,4-thiadiazol.

Die erfindungsgemäßen Verbindungen können hergestellt werden, indem man ein 2-Methyl-5-(2-hydroxy-styryl)-1,3,4-thiadiazol der allgemeinen Formel (II)

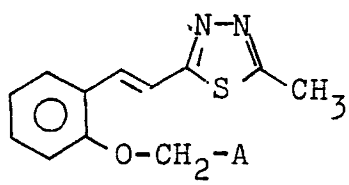

(II)

in der A den Rest

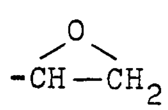 oder 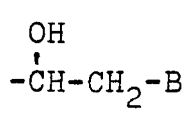

wobei B für eine nukleofuge Abgangsgruppe steht, bedeutet, mit einem Amin der allgemeinen Formel (III)

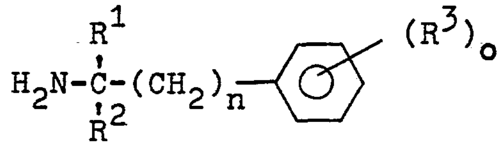 (III),

in der $R^1$, $R^2$, $R^3$, n und o die für Formel I angegebenen Bedeutungen haben, zweckmäßigerweise in einem Lösungsmittel und gegebenenfalls in Gegenwart eines säurebindenden Mittels in an sich bekannter Weise umsetzt und die erhaltene Verbindung gegebenenfalls in das Säureadditionsalz einer physiologisch verträglichen Säure überführt.

Die Abgangsgruppe B stellt bevorzugt ein Halogenatom, insbesondere Chlor, Brom oder Jod, dar. Weiterhin kommen beispielsweise als nukleofuge Abgangsgruppen aromatische oder aliphatische Sulfonsäurereste in Betracht, wie der p-Toluolsulfonsäure-, der p-Brombenzolsulfonsäure- oder der Methansulfonsäurerest.

Die Umsetzungen werden bei Temperaturen von 10 bis 120°C, d.h. bei Raumtemperaturen oder bei höheren Temperaturen, zweckmäßig bei Temperaturen von 50 bis 120°C, durchgeführt. Die Umsetzungen können unter Atmosphärendruck oder in einem geschlossenen Gefäß unter erhöhtem Druck gegebenenfalls unter Erwärmung auf den angegebenen Temperaturbereich durchgeführt werden.

Die Ausgangsverbindungen können direkt, d.h. ohne Zugabe eines Verdünnungs- oder Lösungsmittels, umgesetzt werden. Zweckmäßigerweise werden die Umsetzungen jedoch in Gegenwart inerter Verdünnungs- oder Lösungsmittel, beispielsweise eines niederen Alkohols mit 1 bis 4 C-Atomen, wie Methanol, Äthanol oder Propanol, vorzugsweise Isopropanol oder Äthnol, eines niederen gesättigten Dialkyläthers oder cyclischen Äthers, wie Diäthyläther, 1,2-Dimethoxyäthan, Tetrahydrofuran oder Dioxan, von Benzol oder eines Alkyl-

benzols, wie Toluol oder Xylol, oder eines aliphatischen Kohlenwasserstoffes, wie Hexan, Heptan oder Octan, eines niederen aliphatischen Ketons, wie Aceton, Methyläthylketon oder Methylisobutylketon, eines Dialkylformamids, wie Dimethyl- oder Diäthylformamid, von Dimethylsulfoxid oder in Gegenwart von Wasser oder in Mischungen der genannten Lösungsmittel, durchgeführt.

Bevorzugte Lösungsmittel bei der Umsetzung von 2-Methyl-5-[2-(2,3-epoxypropoxy)-styryl]-1,3,4-thiadiazol mit einem der oben angeführten Amine (III) sind niedere Alkohole, insbesondere Äthanol oder Isopropanol, wobei die Umsetzung bevorzugt bei Temperaturen von 50°C bis 120°C und bei Normaldruck durchgeführt wird. Bei der nukleophilen Substitution eines Restes B sind als Lösungsmittel ein niederes aliphatisches Keton, wie Aceton oder Methyl-isobutylketon, ein cyclischer Äther, insbesondere Tetrahydrofuran oder Dioxan, oder ein Dialkylformamid, wie Dimethylformamid, und Temperaturen von 90° bis 120°C bevorzugt. Gegebenenfalls wird die Reaktion in Gegenwart einer katalytischen Menge Natrium- oder Kaliumjodid durchgeführt.

Es sei erwähnt, daß als Ausgangsverbindungen der Formel II gegebenenfalls auch eine Mischung eines Epoxids mit einem Halogenhydrin in Betracht kommt, da bei der technischen Herstellung der Ausgangsverbindung der Formel II derartige Gemische unter Umständen entstehen können.

Bei einer zweckmäßigen Ausführungsform zur nukleophilen Substitution des Restes B durch das verwendete Amin wird die Umsetzung in Gegenwart einer Base als säurebindendes Mittel durchgeführt. Bevorzugte Base sind Alkalimetallhydroxide, -carbonate, -hydrogencarbonate, -alkoholate oder ein tertiäres organisches Amin, wie Pyridin oder ein Trialkylamin, wie Trimethylamin oder Triäthylamin.

Von den Alkaliverbindungen kommen insbesondere die des Natriums oder Kaliums in Betracht. Dabei wird die Base in stöchiometrischer Menge oder in geringem Überschuß verwendet. Gegebenenfalls ist es zweckmäßig, das zur Umsetzung verwendete Amin III in überschüssiger Menge gleichzeitig als säurebindendes Mittel zu verwenden.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z.B. durch Filtration oder Abdestillieren des Verdünnungs- oder Lösungsmittels aus dem Reaktionsgemisch. Eine Reinigung der erhaltenen Verbindung erfolgt in üblicher Weise, beispielsweise durch Umkristallisieren aus einem Lösungsmittel, Überführen in eine Säureadditionsverbindung oder durch Säuenchromatographie.

Die Ausgangsverbindungen der Formel (II) können durch Alkylierung des 2-Methyl-5-(2-hydroxystyryl)-1,3,4-thiadiazols, das durch Kondensation von Salicylaldehyd mit 2,5-Dimethyl-1,3,4-thiadiazol hergestellt werden kann, mit einem Epihalogenhydrin oder einem $\alpha,\omega$-Dihalogen-2-propanol erhalten werden. Als Epihalogenhydrine kommen Epichlorhydrin, Epobromhydrin und Epijodhydrin und als $\alpha,\omega$-Dihalogen-2-propanol kommen insbesondere 1,3-Dichlor-2-propanol und 1,3-Dibrom-2-propanol in Betracht.

Die erfindungsgemäßen Verbindungen der Formel (I) können aufgrund ihrer Kohlenstoff-Kohlenstoff-Doppelbindung als cis-trans-Isomerengemische vorliegen, die durch bekannte physikalisch-chemische Methoden, beispielsweise durch fraktionierende Kristallisation, Chromatographie oder Sublimation, aufgetrennt werden können.

Die erfindungsgemäßen Verbindungen der Formel (I) besitzen am Kohlenstoffatom 2 der aliphatischen Aminopropanolseitenkette ein Chiralitätszentrum und werden als Racemate erhalten, die durch bekannte Methoden, beispielsweise durch Bildung diastereomerer Salze mit optische aktiven Hilfssäuren, wie Dibenzyolweinsäure, Campher-10-sulfonsäure, Ditoluylweinsäure oder 3-Bromcampher-8-sulfonsäure, in die optisch aktiven Antipoden getrennt werden können.

In einigen Fällen weisen die erfindungsgemäßen Verbindungen der Formel (I) je nach Auswahl der verwendeten Amine (III) ein zweites asymmetrisches Kohlenstoffatom auf und können dann als Diastereomerengemische vorliegen, das in bekannter Weise mit physikalisch-chemischen Methoden in Diastereomerenpaare getrennt werden kann. Optisch reine Formen der erfindungsgemäßen Verbindungen (I) mit zwei Chiralitätszentren sind bei Verwendung von optisch aktiven Aminen der allgemeinen Formel (II) und anschließender Trennung der beiden Diastereomeren, beispielsweise durch fraktionierende Kristallisation oder Chromatographie, erhältlich.

Gegebenenfalls werden die erhaltenen erfindungsgemäßen Verbindungen in das Säureadditionssalz einer physiologisch verträglichen Säure überführt. Als übliche physiologisch verträgliche organische oder anorganische Säuren kommen beispielsweise in Betracht Salzsäure, Bromwasserstoffsäure, Phosphorsäure oder Schwefelsäure, und als organische Säuren beispielsweise Maleinsäure, Fumarsäure, Milchsäure, Weinsäure, Apfelsäure, Zitronensäure, Salicylsäure, Adipinsäure oder Benzoesäure oder können aus Fortschritte der Arzneimittelforschung, Band 10, Seiten 224-225, Birkhäuser Verlag, Basel und Stuttgart, 1966, oder aus Journal of Pharmaceutical Sciences, Volume 66, 1-5 (1977), entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Äthanol oder Propanol, oder einem niederen Keton, wie Aceton, Methyläthylketon oder Methylisobutylketon, oder einem Äther, wie Diäthyläther, Tetrahydrofuran oder Dioxan, erhalten. Zur besseren Kristallabscheidung können auch Mischungen der genannten Lösungsmittel verwendet werden. Darüber hinaus können pharmazeutisch vertretbare wäßrige Lösungen von Säure-Additionsverbindungen der Aminopropanol-Derivate der allgemeinen Formel (I) durch Auflösen der freien Basen der allgemeinen Formel (I) in einer wäßrigen Säurelösung hergestellt werden.

Die erfindungsgemäßen Verbindungen und ihre physiologisch verträglichen Säureadditionssalze weisen wertvolle pharmakologische Eigenschaften auf und sind als Pharmaka mit ß-sympatholytischer Wirkung zur Behandlung der coronaren Herzkrankheit (Angina pectoris), der Hypertonie und von Herzrhythmusstörungen geeignet.

Die ß-sympatholytische Wirkung wurde an Katzen getestet. Als Vergleichssubstanz diente das bekannte ß-Sympatholytikum Propranolol. Zur Prüfung wurden folgende Methoden verwendet:

1. ß-sympatholytische Wirkung
   Isoproterenol (1 µg/kg i.v.) verursacht an narkotisierten Katzen (Gewicht: 2-4kg) Herzfrequenzsteigerungen um durchschnittlich 40 %. ß-Sympatholytika hemmen diese Tachycardien. Isoproterenol wurde vor, sowie 10 und 40 min nach der i.v. Applikation der Prüfsubstanzen appliziert. Zwischen deren Logarithmen

der applizierten Dosen (mg/kg) der Prüfsubstanzen und der Hemmung der Isoproterenol-Tachycardie (%) bestehen lineare Beziehungen. Aus diesen Beziehungen werden als ED 50 % die Dosen ermittelt, welche die Isoproterenol-Tachycardie um 50 % hemmen.

2. Akute Toxizität
Zur Bestimmung der mittleren letalen Dosis (LD 50) werden die Substanzen weiblichen NMRI-Mäusen (Gewicht: 19-26 g) intraperitoneal appliziert.

Die erfindungsgemäßen Substanzen zeichnen sich durch eine hohe ß-sympatholytische Aktivität aus (Tab.). Die für die pharmakotherapeutische Anwendung bedeutsam cardialen $ß_1$—Rezeptoren werden bereits durch Dosen blockiert, die 5,6 (Beispiel 2) bzw. 6,7 ml (Beispiel 4) kleiner sind als die der Vergleichssubstanz Propranolol. Aus dieser hohen Wirksamkeit (ED 50 %) und den letalen Dosen (LD 50), die in der gleichen Größenordnung liegen wie die des Propanolols, ergibt sich eine vergleichsweise 2,6 (Beispiel 4) bzw. 4,2 mal (Beispiel 2) größere therapeutische Breite.

Tabelle

ß-sympatholytische Wirkung und akute Toxizität

| Substanz | $ß_1$-sympatholytische Wirkung 1) | | Akute Toxizität | Therapeutische |
|---|---|---|---|---|
| | ED 50 % 2) | R.W. 3) | LD 50 4) | Breite 5) |
| Bsp. 2 | 0,025 | 5,64 | 81,3 | 3 252 |
| " 4 | 0,021 | 6,71 | 41,7 | 1 986 |
| Propranolol | 0,141 | 1,00 | 108 | 766 |

1) Hemmung der Isoproterenol-Tachycardie. Katze. Hexobarbital-Narkose. Appl.: i.v.

2) Dosis (mg/kg, welche die Isoproterenol-Tachycardie um 50 % hemmt.

3) Relative Wirksamkeit. Propranolol = 1,00.

4) Maus. Appl.: i.p.

5) $\frac{\text{LD 50}}{\text{ED 50 \%}}$

6) Propranolol = 1,00.

Gegenstand der vorliegenden Erfindung sind demnach auch therapeutische Mittel oder Zubereitungen, die neben üblichen Träger- und Verdünnungsmitteln eine Verbindung der Formel (I) als Wirkstoff enthalten, sowie die Verwendung der neuen Verbindungen zu therapeutischen Zwecken.

Die therapeutischen Mittel bzw. Zubereitungen werden mit den üblichen flüssigen oder festen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Calciumcarbonat, Calciumphosphat oder Milchzucker, Sprengmittel wie Mais, Stärke oder Alginsäure, Bindemitteln wie Stärke oder Gelatine, Gleitmittel wie Magnesiumstearat oder Talk und/oder Mitteln zur Erzielung des Depoteffektes wie Carboxypolymethylen, Carboxymethylcellulose, Celluloseacetatphthalat oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können. Lösungen oder Suspensionen mit den erfindungsgemäßen Wirkstoffen können zusätzlich geschmacksverbessernde Mittel wie Saccharin, Cyclamat oder Zucker sowie z.B. Aromastoffe wie Vanillin oder Orangenextrakt, enthalten. Sie können außerdem Suspendierhilfsstoffe, wie Natriumcarboxymethylcellulose oder

Konservierungsstoffe, wie p-Hydroxybenzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln, wie Neutralfetten oder Polyäthylenglykol bzw. deren Derivaten, herstellen.

Als Einzeldosis der erfindungsgemäßen Verbindungen kommen am Menschen 1 bis 100 mg, vorzugsweise 3 bis 50 mg, in Betracht.

Die vorliegende Erfindung wird durch die nachfolgenden Ausführungsbeispiele näher erläutert.

I. Herstellung von Ausgangsverbindungen

Verbindung 1

2-Methyl-5-(2-hydroxy-styryl)-1,3,4-thiadiazol

570 g (5 Mol) 2,5-Dimethyl-1,3,4-thiadiazol und 275 g (2,5 Mol) Salicylaldehyd werden gemischt und unter Durchleiten von Stickstoff langsam auf 150°C erhitzt. Das Gemisch wird 30 h bei 150°C gehalten, dann abgekühlt und nach Abdestillieren von überschüssigem 2,5-Dimethyl-1,3,4-thiadiazol aus Methylglykol umkristallisiert. Man erhält 304 g gelbe Kristalle (56 % d.Th.) vom Fp. 253 bis 254°C.

$C_{11}H_{10}N_2OS$ (218)

| | | | | | | |
|---|---|---|---|---|---|---|
| ber. | C | 60,6 | H | 4,6 | N | 12,8 |
| gef. | C | 59,8 | H | 4,6 | N | 12,4 |

Verbindung 2

2-Methyl-5-[2-(2,3-epoxypropoxy)-styryl]-1,3,4-thiadiazol

3,72 g (0,085 Mol) 55%iges Natriumhydrid in Paraffinöl werden in 150 ml abs. Tetrahydrofuran suspendiert und anschließend tropfenweise mit 18,6 g (0,085 Mol) 2-(2-Hydroxystyryl)-5-methyl-1,3,4-thiadiazol in 200 ml abs. Hexamethylphosphortriamid bei 0 bis 3°C innerhalb von 1,5 h versetzt. Bei Raumtemperatur wird 1 h nachgerührt, anschließend werden 11,7 g (0,085 Mol) Dibromhydrin zugetropft. Man läßt 16 h bei Raumtemperatur stehen. Die Lösung wird auf 1,5 l Eiswasser und 0,5 l gesättigte Natriumchloridlösung gegeben. Der angefallene Feststoff wird abgesaugt und aus Aceton umkristallisiert. Man erhält 11,8 g (51 % d.Th.) gelbe Kristalle. Fp 134 bis 135°C.

$C_{14}H_{14}N_2O_2S$ (274)

| | | | | | | | | | | |
|---|---|---|---|---|---|---|---|---|---|---|
| ber. | C | 61,3 | H | 5,1 | O | 11,7 | S | 11,7 | N | 10,2 |
| gef. | C | 61,3 | H | 5,4 | O | 13,5 | S | 10,5 | N | 8,4 |

Verbindung 3

2-Methyl-5-[2-(2-hydroxy-3-chlor-propoxy)-styryl]-1,3,4--thiadiazol

1 g 2-Methyl-5-[2-(2,3-epoxypropoxy)-styryl]-1,3,4-thiadiazol wird in einem Gemisch aus 100 ml Äthanol und 5 ml einer 3 normalen ätherischen Chlorwasserstoff-Lösung 12 Stunden stehengelassen. Der gebildete Niederschlag wird abgesaugt, mit Äther neutral gewaschen und mit Chloroform über Kiesel-

gel chromatographiert. Die Produkteluate erbringen nach dem Eindampfen zur Trockene spektroskopisch reines 2-Methyl-5--[2-(2-hydroxy-3-chlor-propoxy)-styryl]-1,3,4-thiadiazol vom Schmp. 168 bis 170°C.

1H-NMR-Spektrum ($CDCl_3$, TMS intern)

= 2,5-3,3 (m,6H), 4,8 (s,1H), 5,5-6,0 (m,3H und OH), 6,1-6,3 (m,2H), 7,3 (s,3H)

II. Herstellung von erfindungsgemäßen Verbindungen

(Die in den nachfolgenden Beispielen beschriebenen isolierten Verbindungen sind stets trans-konfigurierte Styryl--Derivate.)

Beispiel 1:

6,0 g 2-Methyl-5-[2-(2,3-epoxypropoxy)-styryl]-1,3,4-thiadiazol und 4,2 g 2-(3,4-Dimethoxyphenyl)-1-methyläthylamin werden in 50 ml Isopropanol 6 Stunden auf Rückflußtemperatur erhitzt. Der nach dem Abdestillieren des Lösungsmittels verbleibende Rückstand wird über eine Kieselgel-Trockensäule (ca. 500 g Kieselgel/50 cm Säulenlänge) mit Chloroform chromatographiert. Der Eindampfrückstand der Produkteluate ergibt 3,1 g (28,9 % d.Th.) 2-Methyl-5-{2-[2-hydroxy--3-(3-(3,4-dimethoxyphenyl)-1-methyläthylamino)-propoxy]--styryl}-1,3,4-thiadiazolmonohydrat vom Schmp. 126 bis 127°C.

$C_{25}H_{31}N_3O_4S \cdot H_2O$ (487,62)

| | | | | | | |
|---|---|---|---|---|---|---|
| ber. | C | 62,6 | H | 6,7 | N | 8,8 |
| gef. | C | 62,9 | H | 6,6 | N | 8,6 |

Die in der nachfolgenden Tabelle angegebenen Verbindungen werden in gleicher Weise aus 2-Methyl-5-[2-(2,3-epoxy-propoxy)-styryl]-1,3,4-thiadiazol und den entsprechenden Aminen erhalten. Sämtliche Verbindungen sind durch Elementaranalysen und $^{1}$HNMR-Spektren charakterisiert.

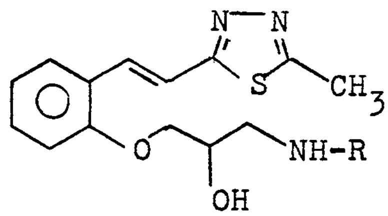

| Nr. | R | Salzform | Schmp. (°C) |
|---|---|---|---|
| 2 | 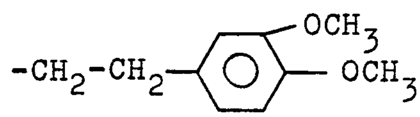 | freies Amin | 131 - 132 |
| 3 | 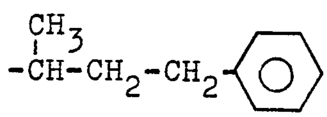 | freies Amin | 114 - 115 |
| 4 | 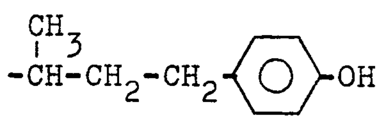 | HCl.$H_2O$ | 169 - 171 |
| 5 | 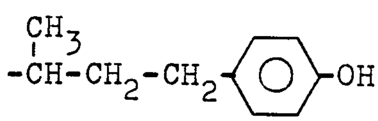 | Oxalsäure | 170 - 172 |
| 6 | 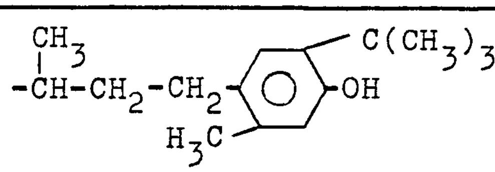 | freies Amin | 156 - 160 |
| 7 | 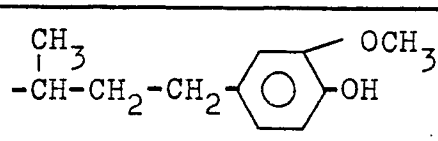 | freies Amin | 148 - 150 |
| 8 | 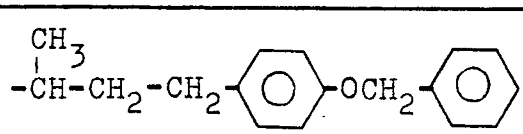 | freies Amin | 112 - 115 |

| Nr. | R | Salzform | Schmp. (°C) |
|---|---|---|---|
| 9 | 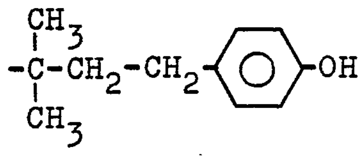 | 2 HCl.$H_2O$ | 140 - 142 |
| 10 | 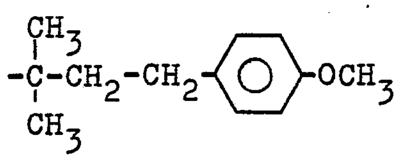 | 2 HCl.2 $H_2O$ | 134 - 136 |
| 11 | 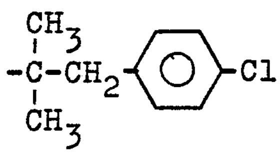 | 2 HCl.2 $H_2O$ | 166 - 168 |
| 12 | 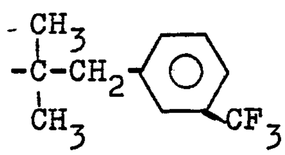 | freies Amin | 106 - 107 |
| 13 | 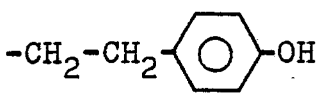 | freies Amin 1/2 $H_2O$ | 177 - 178 |
| 14 | 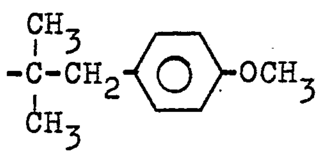 | 2 HCl | 188 - 189 |
| 15 | 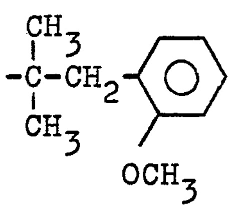 | 2 HCl | 198 - 199 |

III. Formulierungsbeispiele, die in üblicher Weise hergestellt werden:

1. Tabletten:

a)

| | |
|---|---|
| Ein Wirkstoff der Formel I | 5 mg |
| Lactose | 200 mg |
| Methylcellulose | 15 mg |
| Maisstärke | 50 mg |
| Talkum | 11 mg |
| Magnesiumstearat | 4 mg |
| | 285 mg |

b)

| | |
|---|---|
| Ein Wirkstoff der Formel I | 20 mg |
| Lactose | 178 mg |
| Avicel | 80 mg |
| Polywachs 6000 | 20 mg |
| Magnesiumstearat | 2 mg |
| | 300 mg |

c)

| | |
|---|---|
| Ein Wirkstoff der Formel I | 50 mg |
| Polyvinylpyrrolidon (mittl. M.G. 25 000) | 170 mg |
| Polyäthylenglykol (mittl. M.G. 4 000) | 14 mg |
| Hydroxypropylmethylcellulose | 40 mg |
| Talkum | 4 mg |
| Magnesiumstearat | 2 mg |
| | 280 mg |

Der Wirkstoff wird mit Polyvinylpyrrolidon in 10%iger wäßriger Lösung befeuchtet, durch ein Sieb mit der lichten Maschenweite 1,0 mm getrieben und bei 50°C getrocknet. Dieses Granulat wird mit Polyäthylenglykol (mittl. M.G. 4 000), Hydroxy-

propylmethylcellulose, Talkum und Magnesiumstearat vermischt und zu Tabletten à 280 mg verpreßt.

2. Beispiel für Dragees:

| | |
|---|---|
| Ein Wirkstoff der Formel I | 60 mg |
| Lactose | 90 mg |
| Maisstärke | 60 mg |
| Polyvinylpyrrolidon | 6 mg |
| Magnesiumstearat | 1 mg |
| | 217 mg |

Die Mischung der Wirkstoffsubstanz mit Lactose und Maisstärke wird mit einer 8%igen wäßrigen Lösung des Polyvinylpyrrolidons durch Sieb 1,5 mm granuliert, bei 50°C getrocknet und nochmals durch Sieb 1,0 mm gerieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

3. Kapselformulierung:

| | |
|---|---|
| Ein Wirkstoff der Formel I | 5,0 mg |
| Magnesiumstearat | 2,0 mg |
| Milchzucker | 19,3 mg |

4. Injektionslösung:

| | |
|---|---|
| Ein Wirkstoff der Formel I | 10 mg |
| Natriumchlorid | 9 mg |
| destilliertes Wasser, q.s. auf 1,0 ml | |

Patentansprüche

1. Verbindungen der allgemeinen Formel (I),

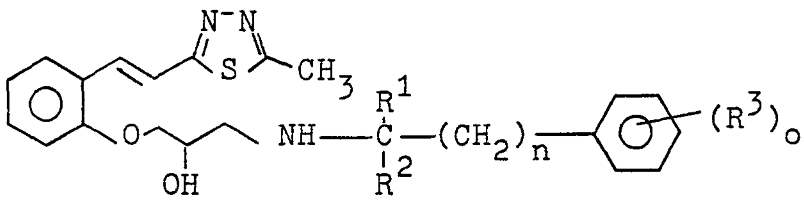

(I),

in der n die Zahlen 2 oder 3, o die Zahlen 1 bis 3, $R^1$ und $R^2$ ein Wasserstoffatom oder eine geradkettige oder verzweigte Alkylgruppe mit 1 bis 5 C-Atomen, $R^3$ ein Wasserstoffatom, ein Halogenatom, eine Hydroxygruppe, eine Alkyl-, Alkoxy- oder Alkylthiogruppe mit 1 bis 5 C-Atomen, gegebenenfalls jeweils in der Alkylgruppe 1- bis 3-fach durch Halogenatome oder einfach durch eine Hydroxy- oder Alkoxygruppe mit 1 bis 3 C-Atomen substituiert, eine Alkenyl-, Alkinyl-, Alkinyloxy- oder Cycloalkoxygruppe mit jeweils 2 bis 6 Kohlenstoffatomen und 3 bis 8 C-Atomen im Ring, eine Aralkoxygruppe mit 7 bis 9 C-Atomen oder eine Aminogruppe, gegebenenfalls mit ein- oder zweifach mit einer Alkylgruppe mit 1 bis 5 Kohlenstoffatomen substituiert,bedeuten, wobei entsprechend den Bedeutungen von o mehrere Reste $R^3$ gleich oder verschieden sein können, oder $R^3$ bedeutet eine Methylendioxygruppe oder eine Alkylengruppe mit 3 oder 4 Kohlenstoffatomen, und ihre Säureadditionsalze.

2. 2-Methyl-5-[2-[2-hydroxy-3-(2-(3,4-dimethoxyphenyl)-äthylamino)-propoxy]-styryl]-1,3,4-thiadiazol.

3. 2-Methyl-5-[2-[2-hydroxy-3-(3-(4-hydroxyphenyl)-1-methylpropylamino)-propoxy]-styryl]-1,3,4-thiadiazol.

4. Verfahren zur Herstellung von Verbindungen der Formel I nach Anspruch 1, <u>dadurch gekennzeichnet</u>, daß man ein 2-Methyl-5-(2-hydroxy-styryl)-1,3,4-thiadiazol der allgemeinen Formel (II)

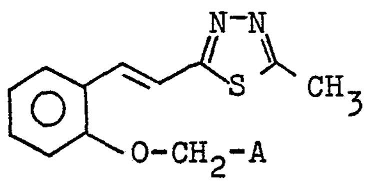 (II)

in der A den Rest

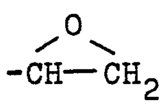 oder 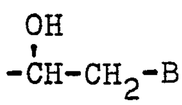

wobei B für eine nukleofuge Abgangsgruppe steht, bedeutet, mit einem Amin der allgemeinen Formel (III)

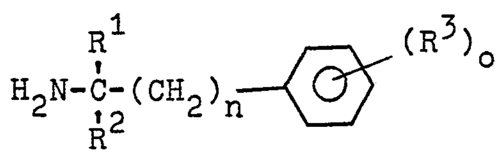 (III),

in der $R^1$, $R^2$, $R^3$, n und o die für Formel I angegebenen Bedeutungen haben, zweckmäßigerweise in einem Lösungsmittel und gegebenenfalls in Gegenwart eines säurebindenden Mittels in an sich bekannter Weise umsetzt und die erhaltene Verbindung gegebenenfalls in das Säureadditionssalz einer physiologisch verträglichen Säure überführt.

5. Therapeutisches Mittel, <u>gekennzeichnet durch</u> einen Gehalt an einer Verbindung der Formel I oder ihres physiologisch verträglichen Säureadditionssalzes als Wirkstoff neben üblichen Trägerstoffen und Verdünnungsmitteln.

0027977

Nummer der Anmeldung

Europäisches Patentamt

# EUROPÄISCHER RECHERCHENBERICHT

EP 80106356.1

| EINSCHLÄGIGE DOKUMENTE | | | KLASSIFIKATION DER ANMELDUNG (Int. Cl.) |
|---|---|---|---|
| Kategorie | Kennzeichnung des Dokuments mit Angabe, soweit erforderlich, der maßgeblichen Teile | betrifft Anspruch | |
| | US - A - 3 598 830 (BERKELHAMMER)<br>* Spalten 1,6,8 *<br>-- | 1 | C 07 D 285/12<br>A 61 K 31/41 |
| | US - A - 3 926 966 (HAGEN)<br>* Spalte 1 *<br>-- | 1 | |
| | US - A - 3 655 661 (WASSON)<br>* Spalten 1,9,10 *<br>---- | 1,4,5 | |
| | | | **RECHERCHIERTE SACHGEBIETE (Int. Cl.)** |
| | | | C 07 D 285/00<br>C 07 D 417/00<br>A 61 K 31/00 |
| | | | **KATEGORIE DER GENANNTEN DOKUMENTE**<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| WIEN | 14-01-1981 | HOCHHAUSER |